# EUROPEAN PATENT SPECIFICATION

(11) **EP 2 024 363 B1**
(45) Date of publication and mention of the grant of the patent: **01.08.2012**
(21) Application number: 07728504.7
(22) Date of filing: 25.04.2007
(51) Int. Cl.: C07D 405/12, A61K 31/444, A61P 25/16, A61P 25/18, A61P 25/22, A61P 25/24

(54) **N-OXIDES OF PYRIDYLMETHYLPIPERAZINE AND -PIPERIDINE DERIVATIVES**
N-OXIDE VON PYRIDYLMETHYLPIPERAZIN- UND PIPERIDINDERIVATEN
N-OXYDES DE DÉRIVÉS DE PYRIDYLMÉTHYLPIPÉRAZINE ET -PIPÉRIDINE

(30) Priority: 02.05.2006 EP 06113393; 02.05.2006 US 796551 P
(43) Date of publication of application: 18.02.2009
(73) Proprietor: Abbott Healthcare Products B.V., 1381 CP Weesp (NL)
(72) Inventor: VAN AAR, Marcel P.M., c/o SOLVAY PHARMACEUTICALS B.V., 1381 CP Weesp (NL); VAN AMSTERDAM, Peter H., c/o SOLVAY PHARMACEUTICALS B.V., 1381 CP Weesp (NL); BARF, Gerrit A., c/o SOLVAY PHARMACEUTICALS B.V., 1381 CP Weesp (NL); BAKKER, Johan Antoine, c/o SOLVAY PHARMACEUTICALS B.V., 1381 CP Weesp (NL); DEN BESTEN, Cathaline, c/o SOLVAY PHARMACEUTICALS B.V., 1381 CP Weesp (NL); HERREMANS, Arnoldus H.J., c/o SOLVAY PHARMACEUTICALS B.V., 1381 CP Weesp (NL); ZORGDRAGER, Jan, c/o SOLVAY PHARMACEUTICALS B.V., 1381 CP Weesp (NL)
(74) Representative: Verhage, Marinus
(86) International application number: PCT/EP2007/054048
(87) International publication number: WO 2007/128694

(56) References cited:
- EP-A1- 0 908 458

## Description

### BACKGROUND OF THE INVENTION

Psychotropic pyridylmethylpiperazine and -piperidine derivatives are disclosed in EP 0 908 458. Among other compounds, the patent discloses 3-[[4-(2,3-dihydro-1,4-benzodioxin-5-yl)-1-piperazinyl]methyl]-5-(4-fluorophenyl)pyridine, also known as SLV313, a dopamine-D₂ receptor antagonist and a serotonin 5-HT_{1A} receptor agonist, in clinical trials as an atypical antipsychotic. Metabolism studies in humans, revealed that one of the main metabolites of SLV313 is its pyridine-N-oxide. This was surprising because this N-oxide could not be demonstrated in the plasma of rats and dogs dosed with the compound in toxicological studies. The piperazine-N-oxide of SLV313 has not been detected as a metabolite of the compound in man nor in any of the animal species used in toxicology studies.

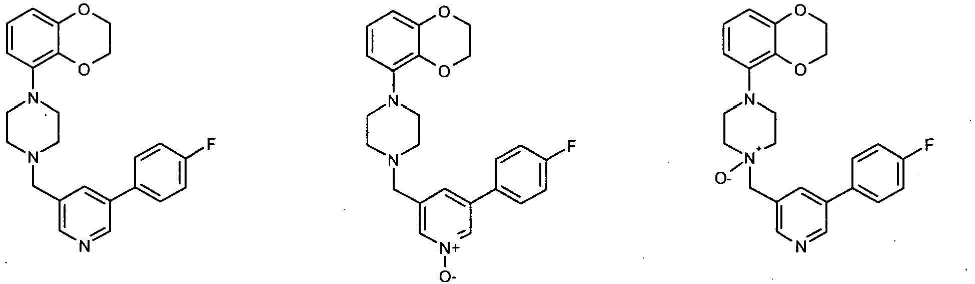

### 3-[[4-(2,3-dihydro-1,4-benzodioxin-5-yl)-1-piperazinyl]methyl]-5-(4-fluorophenyl)-pyridine, 1-(2,3-dihydro-1,4-benzodioxin-5-yl)-4-[[5-(4-fluorophenyl)-1-oxido-3-pyridinyl]methyl]-piperazine 1-(2,3-dihydro-1,4-benzodioxin-5-yl)-4-[[5-(4-fluorophenyl)-3-pyridinyl]-methyl]-4-oxido-piperazine

### (respectively SL V313, its pyridine-N-oxide, and its piperazine-N-oxide)

N-oxides are known since 1894. By now it is very well known that N-oxides are metabolites of many tertiary amines, and in most cases are also intermediates between tertiary amines and their N-dealkylated analogs. Most, but not all, tertiary amine drugs give rise to N-oxides. This is for instance the case with morphine, imipramine, promazine, cinnarizine and nicotine, to name just a few. How much N-oxidation takes place varies from trace amounts to a near quantitative conversion. Some N-oxides were shown to be more potent than their corresponding tertiary amines. The most famous example of these is chlordiazepoxide (Librium^{®}), one of the most frequently used drugs in psychiatric and general medicine. In many more cases however, N-oxides were found to be less potent than their corresponding tertiary amines, and N-oxidation is most commonly regarded to be metabolic deactivation. Whilst N-oxides are easily reduced to their corresponding tertiary amines by chemical means, in the human body this happens to varying degrees. Some N-oxides undergo nearly quantitative reductive conversion to the corresponding tertiary amines and in other cases the conversion is a mere trace reaction or even completely absent *(Bickel,* 1969). Thus, the formation of N-oxides and their corresponding tertiary amines is unpredictable. Once formed, N-oxides may be *more active* than their corresponding tertiary amines, less *active* or even completely *inactive.* N-oxides may be reduced to the corresponding tertiary amines or not. When they are, the reaction may be a mere trace or nearly quantitative.

Since Paracelsus *('Sola dosis facit venenum')* it is generally accepted that therapeutic as well as toxic effects of drugs are related to their concentration at the relevant target sites. Because generally speaking the latter are not easily accessible, blood plasma levels are used as approximations of relevant drug concentrations. During drug development a window of suitable plasma concentrations are defined providing a lower limit or range for efficacy, and an upper range at which side effects start to become apparent. In ideal situations the two concentrations are so far apart that it is easy to administer the drug in such a way that it is effective, yet does not give rise to side effects. In reality, situations are hardly ever ideal, and most drugs show side effects. In most cases the occurrence of side effects can be linked to peak plasma concentrations exceeding the lower level associated with the occurrence of side effects. When given orally in standard formulations, SLV313 produces peak plasma concentrations resulting in some unwanted side effects. This phenomenon, due to peak plasma concentrations observed after oral dosing, was quite unexpected because it does not occur in any of the animal species used in toxicological studies. The problem can be overcome by special dose regimens or by sophisticated slow release formulations of SLV313. Another solution can be a different compound. One with an identical pharmacological profile, but with a much more favourable pharmacokinetic profile.

### DESCRIPTION OF THE INVENTION

*In vitro,* pyridine-N-oxides of SLV313 or its analogs are approximately equipotent with their parent compounds, and, dependent on the route of administration, also *in vivo* they offer the same therapeutic possibilities as disclosed for these compounds (EP 0 908 458). N-oxides formed by oxidation of the tertiary N-atoms of piperazine or piperidine rings are virtually inactive *in vitro.* But upon oral administration they act as prodrugs: They are rapidly converted to their parent compounds.

The present invention relates to :
N-oxides of pyridylmethylpiperazine derivatives selected from 1-(2,3-dihydro-1,4-benzodioxin-5-yl)-4-[[5-(4-fluorophenyl)-1-oxido-3-pyridinyl]methyl]-piperazine and 4-(2,3-dihydro-benzo[1,4]dioxin-5-yl-1-[5-4-fluoro-phenyl]-1-oxy-pyridin-3-ylmethyl)piperazine-1-oxide and tautomers, stereoisomers, pharmacologically acceptable salts, hydrates and solvates thereof.
N-oxides of the present invention may be substantially free of 3-[[4-(2,3-dihydro-1,4-benzodioxin-5-yl)-1-piperazinyl]-methyl]-5-(4-fluorophenyl) pyridine.

The invention relates to racemates, mixtures of diastereomers and the individual stereoisomers of the compounds of claim 1, as well as to hydrates and solvates thereof. Pharmaceutically acceptable salts may be obtained using standard procedures well known in the art, for example by mixing a compound of the present invention with a suitable acid, for instance an inorganic acid or an organic acid.

1-(2,3-dihydro-1,4-benzodioxin-5-yl)-4-[[5-(4-fluorophenyl)-1-oxido-3-pyridinyl]methyl]-piperazine and 4-(2,3-dihydro-benzo[1,4]dioxin-5-yl-1-[5-4-fluoro-phenyl]-1-oxy-pyridin-3-ylmethyl)piperazine-1-oxide respectively the 'pyridine N-oxide' and the 'bis-N-oxide' of 3-[[4-(2,3-dihydro-1,4-benzodioxin-5-yl)-1-piperazinyl]methyl]-5-(4-fluorophenyl) pyridine (SLV313), represented by the formulae:

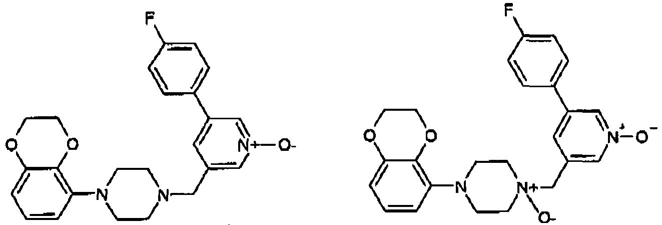

N-oxides of the compounds of the invention of claim 1, as well as the pharmacologically acceptable salts thereof, have dopamine-D₂ receptor antagonistic and 5-HT_{1A} receptor agonistic activity. They are useful in treating Parkinson's disease, aggression, anxiety disorders, autism, vertigo, depression, disturbances of cognition or memory, and in particular schizophrenia, and other psychotic disorders.

The invention also embraces:
pharmaceutical compositions for treating, for example, a disorder or condition treatable by antagonizing dopamine-D₂ receptors and/or activating 5-HT_{1A} receptors, the compositions comprising an N-oxide of a compound of claim 1 or a pharmaceutically acceptable salt thereof, and a pharmaceutically acceptable carrier;
pharmaceutical compositions for treating, for example, a disorder or condition selected from the group consisting of Parkinson's disease, aggression, anxiety disorders, autism, vertigo, depression, disturbances of cognition or memory, and in particular schizophrenia, and other psychotic disorders;
pharmaceutical compositions for treating Parkinson's disease, aggression, anxiety disorders, autism, vertigo, depression, disturbances of cognition or memory, and in particular schizophrenia, and other psychotic disorders, the compositions comprising an N-oxide of a compound of claim 1 or a pharmaceutically acceptable salt thereof, and a pharmaceutically acceptable carrier.

The invention also provides the use of an N-oxide of a compound according to claim 1, or a salt thereof, for the manufacture of medicament.

The invention further relates to combination therapies wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for treating one or more of the conditions listed. Such other therapeutic agent(s) may be administered prior to, simultaneously with, or following the administration of the compounds of the invention.

The invention also provides compounds, pharmaceutical compositions, kits and methods for treating Parkinson's disease, aggression, anxiety disorders, autism, vertigo, depression, disturbances of cognition or memory, and in particular schizophrenia, and other psychotic disorders, the method comprising administering to a patient in need of such treating an N-oxide of a compound of claim 1 or a pharmaceutically acceptable salt thereof.

The compounds of the invention possess dopamine-D₂ receptor antagonistic and 5-HT_{1A} receptor agonistic activity. The (ant)agonizing activities of the compounds of the invention is readily demonstrated, for example, using one or more of the assays described herein or known in the art.

The invention also provides methods of preparing the compounds of the invention and the intermediates used in those methods.

The compounds of the present invention may contain one or more asymmetric centers and can thus occur as racemates and racemic mixtures, single enantiomers, diastereomeric mixtures and individual diastereomers.

Depending on the nature of the various substituents, the molecule can have additional asymmetric centers. Each such asymmetric center will independently produce two optical isomers. All of the possible optical isomers and diastereomers, in mixtures and as pure or partially purified compounds, belong to this invention. The present invention comprehends all such isomeric forms of these compounds. Formula (a) shows the structure of the class of compounds without preferred stereochemistry. The independent syntheses of these diastereomers, or their chromatographic separations, may be achieved as known in the art by appropriate modification of the methodology disclosed therein. Their absolute stereochemistry may be determined by the X-ray crystallography of crystalline products or crystalline intermediates, which are derivatized, if necessary, with a reagent containing an asymmetric center of known absolute configuration. Racemic mixtures of the compounds can be separated into the individual enantiomers by methods well-known in the art, such as the coupling of a racemic mixture of compounds to an enantiomerically pure compound to form a diastereomeric mixture, followed by separation of the individual diastereomers by standard methods, such as fractional crystallization or chromatography. The coupling often consists of the formation of salts using an enantiomerically pure acid or base, for example (-)-di-p-toluoyl-D-tartaric acid and/or (+)-di-p-toluoyl-L-tartaric acid. The diasteromeric derivatives may then be converted to the pure enantiomers by cleavage of the added chiral residue. The racemic mixture of the compounds can also be separated directly by chromatographic methods utilizing chiral stationary phases: Methods well-known in the art. Alternatively, any enantiomer of a compound may be obtained by stereoselective synthesis using optically pure starting materials or reagents of known configuration by methods well-known in the art.

Cis and trans isomers of N-oxides of a compound of claim 1, or a pharmaceutically acceptable salt thereof, also belong to the invention, and this also applies to tautomers of N-oxides of N-oxides of compounds of claim 1, or a pharmaceutically acceptable salt thereof.

Some of the crystalline forms for the compounds may exist as polymorphs: as such intended to belong to the invention. In addition, some of the compounds may form solvates with water (i.e. hydrates), or common organic solvents. Such solvates also fall within the scope of this invention.

Isotopically-labeled N-oxides of a compound of claim 1, or pharmaceutically acceptable salts thereof, including N-oxides of N-oxides of compounds of claim 1, isotopically-labeled to be detectable by PET or SPECT, also fall within the scope of the invention. The same applies to N-oxides of N-oxides of compounds of claim 1, labeled with [¹³C]-, [¹⁴C]-, [³H]-, [¹⁸F]-, [¹²⁵I]- or other isotopically enriched atoms, suitable for receptor binding or metabolism studies.

The chance finding that N-oxide metabolites of certain pyridylmethylpiperazine and - piperidine derivatives are useful as alternatives or as 'prodrugs' of their respective parent compounds, offers possibilities to use these compounds as alternatives, with the clinical benefits of an extended duration of action and a blunted peak plasma concentration, leading to an enhanced side-effect profile. Thus in some embodiments of the present invention, compounds of the present invention may be provided substantially free of parent compound 3-[[4-(2,3-dihydro-1,4-benzodioxin-5-yl)-1-piperazinyl]methyl]-5-(4-fluorophenyl) pyridine (SLV-313). By substantially free is meant that compound of the present invention contains less than about 50%, 40%, 30%, 20%, 10%, 1%, 0.5% or is, within detectable limits, free of 3-[[4-(2,3-dihydro-1,4-benzodioxin-5-yl)-1-piperazinyl]methyl]-5-(4-fluorophenyl) pyridine (SLV-313) as an impurity. Pharmaceutical compositions containing N-oxides of SLV-313 which are substantially free of SLV-313 are envisioned in accordance with the present invention.

### DEFINITIONS OF CHEMICAL AND OTHER TERMS

With reference to substituents, the term **"independently"** means that when more than one of such substituents are possible, they may be the same or different from each other. To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value. Throughout the description and the claims of this specification the word **"comprise"** and variations of the word, such as "comprising" and "comprises" is not intended to exclude other additives, components, integers or steps. The term **"composition"** as used herein encompasses a product comprising specified ingredients in predetermined amounts or proportions, as well as any product that results, directly or indirectly, from combining specified ingredients in specified amounts. In relation to pharmaceutical compositions, this term encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. The pharmaceutical composition includes enough of the active object compound to produce the desired effect upon the progress or condition of diseases. Accordingly, the pharmaceutical compositions of the present invention encompass any composition made by admixing a compound of the present invention and a pharmaceutically acceptable carrier.

Within the context of this application, the term **'combination preparation'** comprises both true combinations, meaning an N-oxide of a compound of formula (a) or a pharmaceutically acceptable salt thereof, and other medicaments physically combined in one preparation such as a tablet or injection fluid, as well as **'kit-of-parts',** comprising an N-oxide of a compound of formula (a) or a pharmaceutically acceptable salt thereof, and SLV313 or another medicament in separate dosage forms, together with instructions for use, optionally with further means for facilitating compliance with the administration of the component compounds, e.g. label or drawings. With true combinations, the pharmacotherapy by definition is simultaneous. The contents of 'kit-of-parts', can be administered either simultaneously or at different time intervals. Therapy being either concomitant or sequential will be dependant on the characteristics of the other medicaments used, characteristics like onset and duration of action, plasma levels, clearance, etc., as well as on the disease, its stage, and characteristics of the individual patient.

By **"pharmaceutically acceptable"** it is meant the carrier, diluent or excipient must be compatible with the other ingredients of the formulation and not deleterious to the recipient thereof. **Dose:** The affinity of the compounds of the invention for dopamine-D₂ and 5-HT_{1A} receptors was determined as described below. From the binding affinity measured for a given compound of formula (a), one can estimate a theoretical lowest effective dose. At a concentration of the compound equal to twice the measured Kᵢ-value, nearly 100% of the receptors likely will be occupied by the compound. By converting that concentration to mg of compound per kg of patient one obtains a theoretical lowest effective dose, assuming ideal bioavailability. Pharmacokinetic, pharmaco-dynamic, and other considerations may alter the dose actually administered to a higher or lower value. The dose of the compound to be administered will depend on the relevant indication, the age, weight and sex of the patient and may be determined by a physician. The dosage will preferably be in the range of from 0.01 mg/kg to 10 mg/kg. The typical daily dose of the active ingredients varies within a wide range and will depend on various factors such as the relevant indication, the route of administration, the age, weight and sex of the patient and may be determined by a physician. In general, oral and parenteral dosages will be in the range of 0.1 to 1,000 mg per day of total active ingredients. The term **"therapeutically effective amount"** as used herein refers to an amount of a therapeutic agent to treat or prevent a condition treatable by administrating a composition of the invention. That amount is the amount sufficient to exhibit a detectable therapeutic, preventative or ameliorative response in a tissue system, animal or human. The effect may include, for example, treating or preventing the conditions listed herein. The precise effective amount for a subject will depend upon the subject's size and health, the nature and extent of the condition being treated, recommendations of the treating physician (researcher, veterinarian, medical doctor or other clinician), and the therapeutics, or combination of therapeutics, selected for administration. Thus, it is not useful to specify an exact effective amount in advance. The term **"pharmaceutically acceptable salt"** refers to those salts that are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well-known in the art. They can be prepared *in situ* when finally isolating and purifying the compounds of the invention, or separately by reacting them with pharmaceutically acceptable non-toxic bases or acids, including inorganic or organic bases and inorganic or organic acids. The term **"treatment"** as used herein refers to any treatment of a mammalian, preferably human condition or disease, and includes: (1) preventing the disease or condition from occurring in a subject predisposed to the disease, but not yet diagnosed as having it, (2) inhibiting the disease or condition, i.e., arresting its development, (3) relieving the disease or condition, i.e., causing the condition to regress, or (4) stopping the symptoms of the disease. As used herein, the term **"medical therapy"** intendeds to include prophylactic, diagnostic and therapeutic regimens carried out *in vivo* or *ex vivo* on humans or other mammals. The term **"subject"** as used herein, refers to an animal, preferably a mammal, most preferably a human, who has been the object of treatment, observation or experiment.

### EXAMPLES

### EXAMPLE 1: ANALYTICAL METHODS

### Liquid Chromatography- Mass Spectrometrry (LC-MS)

The LC-MS system consists of 2 Perkin elmer series 200 micro pumps. The pumps are connected to each other by a 50 µl tee mixer, connected to a Gilson 215 auto sampler. The method is as follows:

| step | total time | flow (µl/min) | A(%) | B(%) |
|---|---|---|---|---|
| 0 | 0 | 2000 | 95 | 5 |
| 1 | 1.8 | 2000 | 0 | 100 |
| 2 | 2.5 | 2000 | 0 | 100 |
| 3 | 2.7 | 2000 | 95 | 5 |
| 4 | 3.0 | 2000 | 95 | 5 |

| | | | | |
|---|---|---|---|---|
| A= 100% Water with 0.025% HCOOH and 10mmol NH4HCOO pH= +/- 3 B= 100% ACN with 0.025% HCOOH | | | | |

The auto sampler has a 2 µl injection loop. The auto sampler is connected to a Waters Atlantis C18 30*4.6 mm column with 3 µm particles. The column is thermo stated in a Perkin Elmer series 200 column oven at 40° C. The column is connected to a Perkin Elmer series 200 UV meter with a 2.7 µl flowcel. The wavelength is set to 254 nm. The UV meter is connected to a Sciex API 150EX mass spectrometer. The mass spectrometer has the following parameters: Scanrange:150-900 a.m.u.; polarity: positive; scan mode: profile; resolution Q1: UNIT ; step size: 0.10 a.m.u.; time per scan: 0.500 sec; NEB: 10; CUR: 10; IS: 5200; TEM: 325; DF: 30; FP: 225 and EP: 10. The light scattering detector is connected to the Sciex API 150. The light scattering detector is a Sedere Sedex 55 operating at 50° C and 3 bar N₂. The complete system is controlled by a G3 powermac.

### EXAMPLE 2: SYNTHESES OF SPECIFIC COMPOUNDS

The specific compounds of which the synthesis is described below are intended to further illustrate the invention in more detail.

Other embodiments of the invention will be apparent to those skilled in the art from consideration of the specification and practice of the invention disclosed herein.

### 1-(2,3-dihydro-1,4-benzodioxin-5-yl)-4-[[5-(4-fluorophenyl)-1-oxido-3-pyridinyl]methyl]-piperazine, compound 1, the 'pyridine N-oxide' of SLV313

The synthesis of 1-(2,3-dihydro-1,4-benzodioxin-5-yl)-4-[[5-(4-fluorophenyl)-1-oxido-3-pyridinyl]-methyl]-piperazine (**1**) is outlined in scheme 1. N-oxidation of pyrifenchloride (**2**) affords pyrifen-chloride-N-oxide (**3**) which is coupled with eltoprazine (**4**) to give the intended N-oxide:

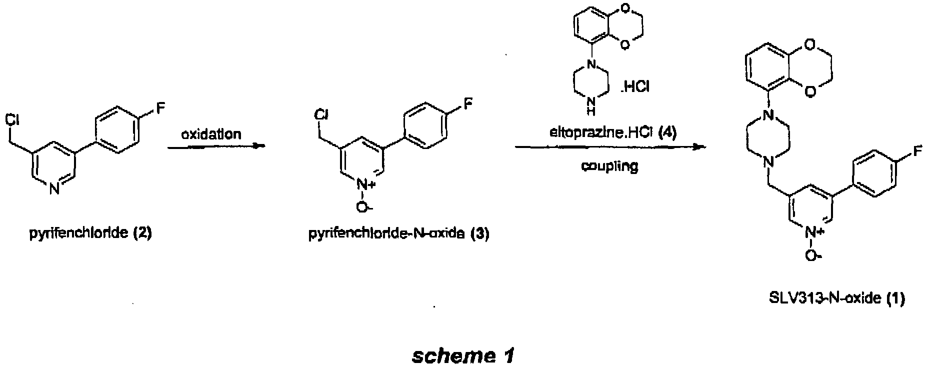

### Step 1: oxidation of pyrifenchloride (2) with H₂O₂:

A mixture of 4.66 g (21.0 mmol) of 3-(chloromethyl)-5-(4-fluorophenyl)pyridine (pyrifenchloride, (**2**), obtainable as described in EP 0 908 458), 70 ml of acetic acid, and 6 ml of 35% H₂O₂ is heated to 70°C. After 95 hours of reaction another portion of 2 ml of 35% H₂O₂ is added. Stirring for another 23 hours at 70°C is followed by the addition of an extra amount of 2 ml of 35% H₂O₂ The reaction mixture is additionally stirred overnight at 70°C.

The reaction mixture is concentrated to a brown oil using a rotary evaporator. The residue is dissolved in 100ml of dichloromethane. Addition of 50 ml of a 10% aqueous sodium carbonate solution is followed by separation of the layers. The organic layer is extracted with 100 ml of dichloromethane and 50 ml of dichloromethane, respectively. The combined organic layers are concentrated under reduced pressure using a rotary evaporator. The crude product is purified using PrepHPLC (Inertsil ODS-3 column, eluens gradient from 10/90% acetonitrile/ water to 90/10% acetonitrile/water containing 0.1% HCOOH) to obtain 1.48 g (29 mmol, 30% yield) of the corresponding N-oxide (**3**).

### Step 1^{a}: oxidation of pyrifenchloride (2) with metachloroperbenzoic acid (mCPBA)

1.11 g of mCPBA is stirred in 20 ml of DCM. 1.0 g of pyrifenchloride (**2**) is dissolved in 20 ml DCM and the yellow solution is added to the m-CPBA solution under stirring at room temperature. The clear yellow solution is stirred for 90 minutes after which another 0.55 g of m-CPBA is added. Stirring is continued for another hour, where after 25 ml water and 1 g NaHCO₃ are added and the reaction mixture is stirred for 10 minutes. The layers are separated and 10 ml water and 2 ml 2N NaOH are added. After stirring, the layers are separated and the water layer is extracted with EtOAc. The combined organic layers are washed with water and concentrated until dry to afford 0.98 g (91% c/c) of the corresponding N-oxide as an orange solid.

On a larger scale, the synthesis proceeds as follows:

192 g (0.745 mole) of 3-(chloromethyl)-5-(4-fluorophenyl)pyridine (**2**) as monohydrochloride salt is suspended in 1 I of DCM. 140 g of NaHCO₃ is added followed by dosing of 2 liter of water for 15 minutes. 239 g of mCPBA (1.25 equivalents) is stirred in 1350 ml of DCM and this solution is added drop wise to the pyrifen chloride suspension in DCM/water for 20 minutes. The clear yellow solution is stirred for 90 minutes. The layers are separated. The water layer is extracted with 335 ml of DCM. The combined organic layers are extracted with a mixture of 1675 ml of water and 670 ml of 2N NaOH. Separation of the layers is followed by extraction of the water layer with 670 ml of DCM. About 2.4 I of DCM is removed by distillation. 2.7 I of MtBE (methyl-tert-butyl ether) is added resulting in crystallization of the product. 2.5 I of DCM/MtBE mixture is distilled of. The mixture is cooled to 5°C during 30 minutes and stirred at that temperature for 30 minutes. The solid material is isolated by filtration to afford 161 g (91 %) of the white crystalline corresponding N-oxide.

### Step 2: coupling of pyrifenchloride N-oxide (3) with eltoprazine (4):

To a suspension of 1.48 g (6.23 mmol) of pyrifenchloride N-oxide (**3**), 1.63 g (1.0 equiv.) of eltoprazine.HCl (**4**), obtainable as described in EP 0 189 612, 1.04 g (1.2 equiv.) of potassium carbonate and 27.5 ml of 2-butanone is added 1.5 ml of water. The mixture is heated to reflux (74°C) for 20 hours. Cooling to room temperature is followed by the addition of 27.5 ml of water. All materials dissolve. The layers are separated. The water layer is extracted twice with 45 ml of 2-butanone, followed by washing of the combined organic layers with 30 ml of water. The combined organic layers are concentrated using a rotary evaporator. The crude product is purified using PrepHPLC (Inertsil ODS-3 column, eluens gradient from 10/90% acetonitrile/water to 90/10% acetonitrile/water with 0.1% HCOOH) to obtain 1.83 g (4.34 mmol, 70% yield) of the corresponding SLV313-N-oxide: 1-(2,3-dihydro-1,4-benzodioxin-5-yl)-4-[[5-(4-fluorophenyl)-1-oxido-3-pyridinyl]-methyl]-piperazine M.p. (DSC): 171°C.

On a larger scale, the synthesis proceeds as follows:

155 ml of water is added to a stirred suspension of 155 g (652 mmole) of N-oxide **3**, 167.5 g (1 equivalent) of eltoprazine.HCl (**4**), 108.2 g (781 mmole) of powdered potassium carbonate and 2.5 l of 2-butanone. The mixture is refluxed (76°C) for 3 hours. The mixture is cooled to about 50°C and is extracted three times with 500 ml of water at this temperature. The combined water layers are extracted with 200 ml of MEK. The organic layers are combined and 1.6 l of 2-butanone is distilled of. The mixture is cooled to 0°C. The solution is seeded at 35°C. The resulting suspension is stirred for 30 minutes at 0°C. The solid material is isolated by filtration to afford 257.3 g (94%) of crude SLV313-N-oxide. Recrystallisation of 480 g of the crude N-oxide from 3.5 l of EtOAc yields 404 g (84 %) of 1-(2,3-dihydro-1,4-benzodioxin-5-yl)-4-[[5-(4-fluorophenyl)-1-oxido-3-pyridinyl]-methyl]-piperazine (compound 1). M.p. (DSC): 171°C.

### %4-(2,3-dihydro-benzo[1,4]dioxin-5-yl-1-[5-4-fluorophenyl]-1-oxy-pyridin-3-ylmethyl)pipera-zine-1-oxide, compound 3, the bis-N-oxide'of SLV313.

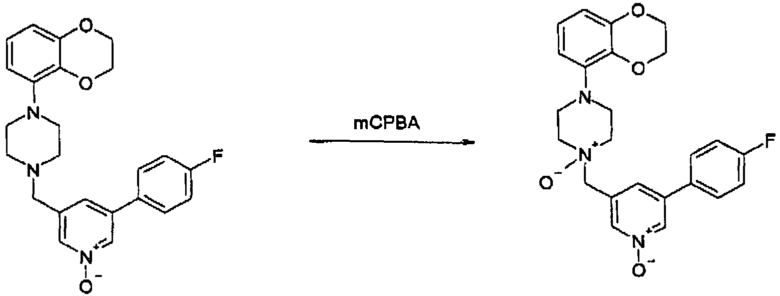

0.5 g (1.19 mmol) 1-(2,3-dihydro-1,4-benzodioxin-5-yl)-4-[[5-(4-fluorophenyl)-1-oxido-3-pyridi-nyl]-methyl]-piperazine, the 'pyridine N-oxide' of SLV313, was suspended in 40 ml acetone. 0.32 g (1.85 mmol) mCPBA was added. The clear solution was stirred for 30 minutes at room temperature. The mixture was concentrated *in vacuo* and the residue was purified by silica gel chromatography (DMA 1) to yield 0.26 g (50%) 4-(2,3-dihydro-benzo[1,4]dioxin-5-yl-1-[5-4-fluorophenyl]-1-oxy-pyridin-3-ylmethyl)piperazine-1-oxide, compound 3. M.p.: 170-173°C.

Similarly, the N-oxides of other compounds having formula (a) can be synthesized.

The selection of the particular synthetic procedures depends on factors known to those skilled in the art such as the compatibility of functional groups with the reagents used, the possibility to use protecting groups, catalysts, activating and coupling reagents and the ultimate structural features present in the final compound being prepared.

### EXAMPLE 3: FORMULATIONS USED IN ANIMAL STUDIES

**For oral *(p.o.)* administration:** to the desired quantity (0.5-5 mg) of the solid test compound in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7. Remaining particles in the suspension were further suspended by using an ultrasonic bath.

**For intravenous *(i.v.)* administration:** compounds were dissolved in physiological saline (0.9% NaCl) and the pH was adjusted to 7.

### EXAMPLE 4: PHARMACOLOGICAL METHODS

***In vitro* affinity for neurotransmitter receptors:** The binding data collected below were either obtained by CEREP (128, rue Danton, 92500 Rueil-Malmaison, France) or at Solvay Pharmaceuticals B.V. (C.J. van Houtenlaan 36, 1381 CP Weesp, The Netherlands), using well documented standard procedures. The affinities for dopamine-D₂ and 5-HT_{1A} receptors for instance, were measured as described by Creese (1997) and Gozlan (1983) respectively.

***In vitro* (ant)agonistic activity** at human D₂ receptors and 5-HT_{1A} receptors was measured according to the methods described by Solomon (1974) and Weiss (1985) respectively, on the formation of adenylate cyclase in CHO cell-lines expressing these cloned receptors.

**Lower lip retraction,** an *in vivo* animal model for serotonin 5-HT_{1A} receptor agonistic activity, was measured according to the method described by Berendsen (1989); **Apomorphine-induced climbing behaviour in mice,** an *in vivo* animal model for dopamine-D_{z} receptor antagonistic activity, was determined according to the method described by Costall (1978).

The N-oxides of the compounds of the invention of the general formula (a), as well as the pharmacologically acceptable salts thereof, are alternatives for the parent compounds or prodrugs thereof, having dopamine-D₂ receptor antagonistic activity combined with 5-HT_{1A} receptor agonistic activity. They are useful in the treatment of affections or diseases of the central nervous system caused by disturbances in either the dopaminergic or serotinergic systems, for example: Parkinson's disease, aggression, anxiety disorders, autism, vertigo, depression, disturbances of cognition or memory and in particular schizophrenia and other psychotic disorders.

*In the following Example, the data of the piperazine N-oxide of SLV313 are presented for comparison.*

### EXAMPLE 5: PHARMACOLOGICAL TESTRESULTS

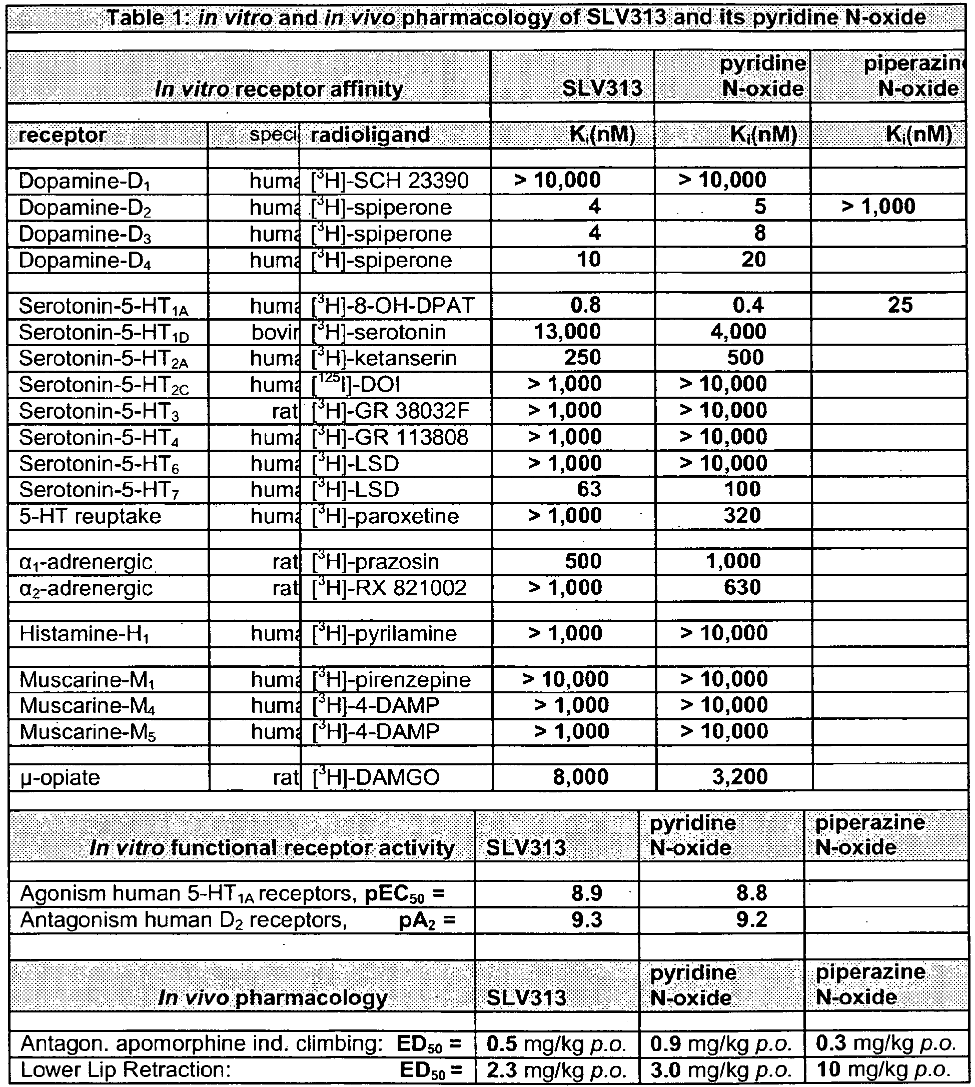

Obviously, the pharmacological profiles, *in vitro* as well as *in vivo,* of SLV313 and its pyridine N-oxide are identical. Relative to those compounds, the piperazine N-oxide is nearly inactive *in vitro,* but equipotent *in vivo.* A prototypical prodrug.

SLV313 and its pyridine N-oxide were individually administered (either intravenously (i.v.) or orally (p.o.)) to mice (3 animals per time point), after which their blood was analyzed by LC-MS (*method see above*) for both SLV313 and its pyridine N-oxide. Data were averaged (n=3), and collected in table 2.

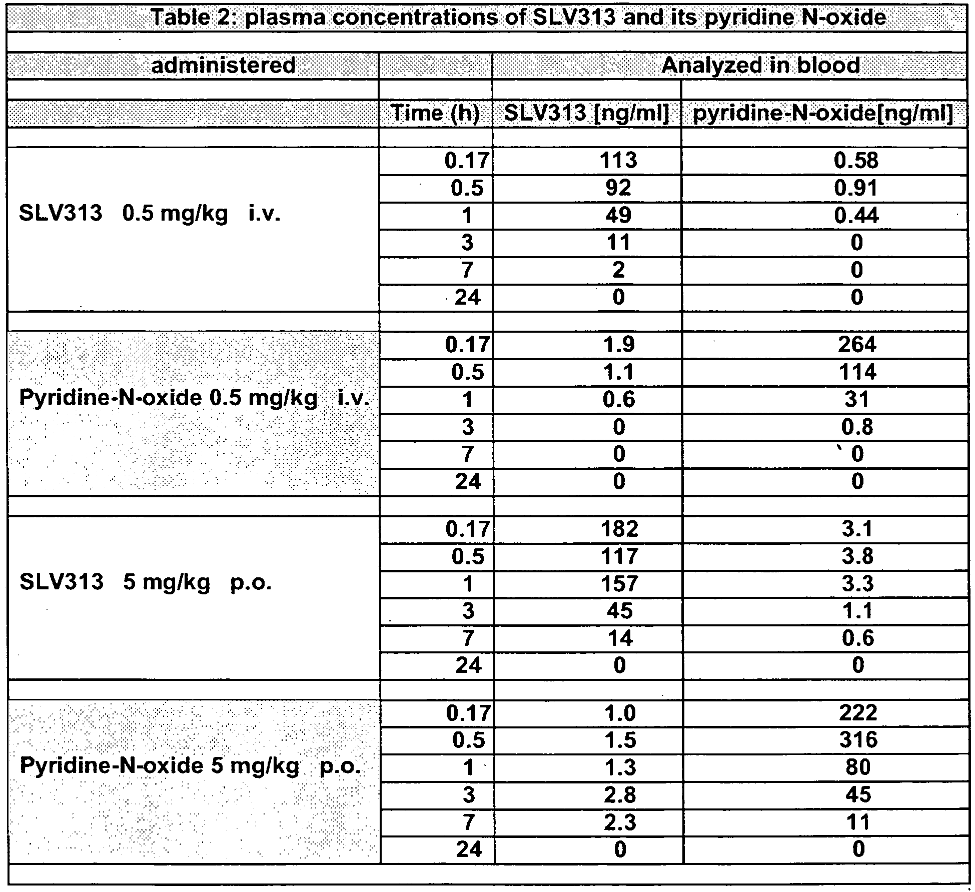

When administered to mice (*i.v.* or p.o.) SLV313 to a marginal extend is metabolized to its pyridine N-oxide: The concentration thereof never exceeds 1 - 2% of that of the parent compound. When the N-oxide itself is administered it is only to a very small extend (less than 1 %) reduced to its parent compound.

In mice, the pyridine N-oxide is an alternative to, rather than a prodrug of, SLV313.

SLV313 and its piperazine N-oxide were individually administered (either intravenously (i.v.) or orally (p.o.)) to mice (3 animals per time point), after which their blood was analyzed by LC-MS (*method see above*) for both SLV313 and its piperazine N-oxide. Data were averaged (n=3), and collected in table 3.

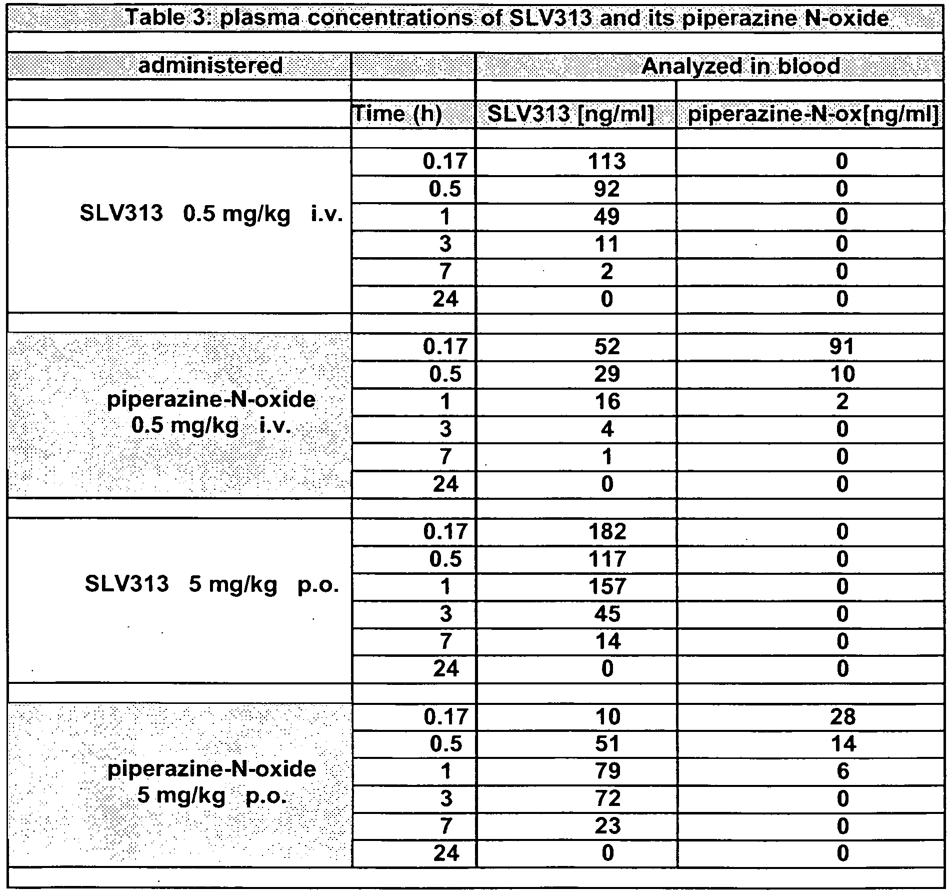

When administered to mice (i.v. or p.o.), SLV313 is not metabolized to its piperazine-N-oxide.

When the piperazine-N-oxide itself is administered, within half an hour the concentration thereof in the plasma exceeds that of the parent molecule. The piperazine-N-oxide evidently is a prodrug of SLV313.

SLV313 and its piperazine N-oxide were individually administered (either intravenously (i.v.) or orally (p.o.)) to mice (3 animals per time point), after which their brain was analyzed by LC-MS (*method see above*) for both SLV313 and its piperazine N-oxide. Data were averaged (n=3), and collected in table 4.

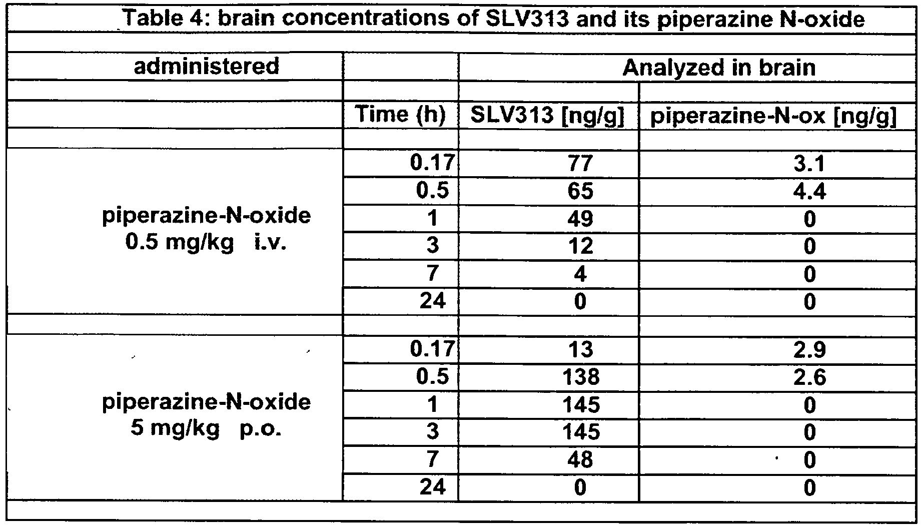

When administered to mice (i.v. or p.o.), the piperazine-N-oxide can hardly be detected in brain,
but the presence of the parent compound SLV313 is evident.

### Volume of distribution

The volume of distribution (V_{D}), also known as the *'apparent* volume of distribution', is a pharmacological term used to quantify the distribution of a drug throughout the body after oral or parenteral dosing. It is defined as the volume in which the amount of drug would need to be uniformly distributed in to produce the observed concentration *(Widmark, 1919).*

The volume of distribution of SLV313 and its pyridine N-oxide was measured in mice, using the standard procedure well known in the art:

| **compound** | **V_{D} in liters per kilogram** | **V_{D} in average person of 70 kg** |
|---|---|---|
| | | |
| SLV313 | **5** | **350 L** |
| SLV313-pyridine-N-oxide | **1** | **70 L** |

From the data given above it is immediately evident that the volume of distribution for SLV313 is five times higher than that of its pyridine N-oxide.

The volume of distribution is not a physiological volume, but rather the ratio between the total amount of drug in the body and its concentration in plasma. It is generally accepted that the volume of distribution is more or less the same across species, making it a parameter that, when measured in laboratory animals, has a high predictive value towards man.

The value of 350 L, calculated for an average person weighing 70 kg, is much larger than that person's body volume, indicating that the compound must be extensively distributed into tissues, leaving low concentrations in the plasma. The pyridine N-oxide of SLV313 has a calculated volume of distribution of 70 L in a person weighing 70 kg, indicating a distribution largely confined to total body water.

### Partition coefficient.

Partition coefficients (log P) of SLV313 and its N-oxides were calculated and determined (at pH 7.0) by methods well known in the art.

| **compound** | **molweight** | **ALog P (calculated)** | **Log P (experimental)** |
|---|---|---|---|
| | | | |
| SLV313 | **405.5** | **3.9** | **3.6** |
| SLV313-pyridine-N-oxide | **421.5** | **3.1** | **2.6** |
| SLV313-piperazine-N-oxide | **421.5** | **3.1** | **1.7** |
| SLV313-bis-N-oxide | **437.5** | **2.2** | |

Evidently, the N-oxides of SLV313 are less lipophilic than their parent compound. When given orally, in equal doses, the N-oxides will enter the bloodstream slower than SLV313, and their peak concentrations will be lower than those observed after SLV313.

The scheme below illustrates the invention: different N-oxides of SLV313, one of the compounds of formula (a). The 'piperazine N-oxide', not found as metabolite, is virtually inactive *in vitro,* but - after oral dosing - rapidly reduced to the parent molecule SLV313. The 'pyridine N-oxide', a metabolite in man, has a pharmacological profile closely matching that of the parent compound. It is an active metabolite.

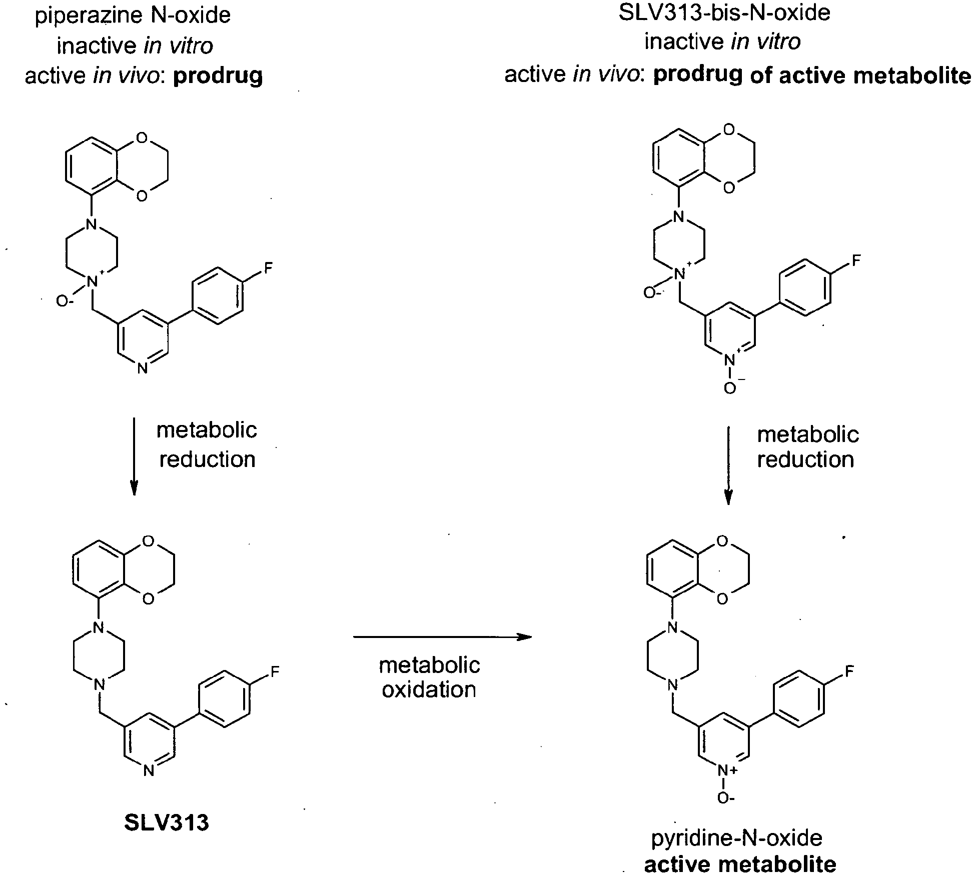

Analogously, the SLV313-bis-N-oxide (the 'piperazine N-oxide of the pyridine N-oxide') can be found inactive *in vitro,* but equipotent with its corresponding parent molecule: the pyridine N-oxide. The 'bis-N-oxide' is a prodrug of an active metabolite.

### EXAMPLE 6: PHARMACEUTICAL PREPARATIONS

For clinical use, N-oxides of compounds of formula (a) are formulated into pharmaceutical compositions that are important and novel embodiments of the invention because they contain the compounds, more particularly specific compounds disclosed herein. Types of pharmaceutical compositions that may be used include, but are not limited to, tablets, chewable tablets, capsules (including microcapsules), solutions, parenteral solutions, ointments (creams and gels), suppositories, suspensions, and other types disclosed herein or apparent to a person skilled in the art from the specification and general knowledge in the art. The compositions are used for oral, intravenous, subcutaneous, tracheal, bronchial, intranasal, pulmonary, transdermal, buccal, rectal, parenteral or other ways to administer. The pharmaceutical formulation contains at least one compound of formula (a) in admixture with a pharmaceutically acceptable adjuvant, diluent and/or carrier. The total amount of active ingredients suitably is in the range of from about 0.1% (w/w) to about 95% (w/w) of the formulation, suitably from 0.5% to 50% (w/w) and preferably from 1% to 25% (w/w).

The compounds of the invention can be brought into forms suitable for administration by means of usual processes using auxillary substances such as liquid or solid, powdered ingredients, such as the pharmaceutically customary liquid or solid fillers and extenders, solvents, emulsifiers, lubricants, flavorings, colorings and/or buffer substances. Frequently used auxiliary substances include magnesium carbonate, titanium dioxide, lactose, saccharose, sorbitol, mannitol and other sugars or sugar alcohols, talc, lactoprotein, gelatin, starch, amylopectin, cellulose and its derivatives, animal and vegetable oils such as fish liver oil, sunflower, groundnut or sesame oil, polyethylene glycol and solvents such as, for example, sterile water and mono- or polyhydric alcohols such as glycerol, as well as with disintegrating agents and lubricating agents such as magnesium stearate, calcium stearate, sodium stearyl fumarate and polyethylene glycol waxes. The mixture may then be processed into granules or pressed into tablets.

The active ingredients may be separately premixed with the other non-active ingredients, before being mixed to form a formulation. The active ingredients may also be mixed with each other, before being mixed with the non-active ingredients to form a formulation.

Soft gelatin capsules may be prepared with capsules containing a mixture of the active ingredients of the invention, vegetable oil, fat, or other suitable vehicle for soft gelatin capsules. Hard gelatin capsules may contain granules of the active ingredients. Hard gelatin capsules may also contain the active ingredients together with solid powdered ingredients such as lactose, saccharose, sorbitol, mannitol, potato starch, corn starch, amylopectin, cellulose derivatives or gelatin. Dosage units for rectal administration may be prepared (i) in the form of suppositories that contain the active substance mixed with a neutral fat base; (ii) in the form of a gelatin rectal capsule that contains the active substance in a mixture with a vegetable oil, paraffin oil or other suitable vehicle for gelatin rectal capsules; (iii) in the form of a ready-made micro enema; or (iv) in the form of a dry micro enema formulation to be reconstituted in a suitable solvent just prior to administration.

Liquid preparations may be prepared in the form of syrups, elixirs, concentrated drops or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder, reconstituted with a suitable solvent prior to use. Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation, reconstituted with a suitable solvent before use.

Also provided according to the present invention are formulations and 'kits of parts' comprising one or more containers filled with one or more of the ingredients of a pharmaceutical composition of the invention, for use in medical therapy. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration. The use of formulations of the present invention in the manufacture of medicaments for use in treating a condition in which antagonism of dopamine-D₂ receptors and/or activation of 5-HT_{1A} receptors is required or desired, and methods of medical treatment or comprising the administration of a therapeutically effective total amount of at least one compound of formula (a), either as such or, in the case of prodrugs, after administration, to a patient suffering from, or susceptible to, a condition in which antagonism of dopamine-D₂ receptors and/or activation of 5-HT_{1A} receptors is required or desired.

### REFERENCES

Berendsen et al., Pharmacol. Biochem. Behav. 33, 821-827, 1989.
Bickel, M.H.,: "The pharmacology and Biochemistry of N-oxides", Pharmacol. Reviews, 21(4), 325 - 355, 1969.
Costall, B., Naylor, R.J. and Nohria, V., 'Differential actions of typical and atypical agents on two behavioural effects of apomorphine in the mouse', Brit J Pharmacol, 63: 381-382, 1978.
Creese I, Schneider R and Snyder S.H., '[3H]-Spiroperidol labels dopamine receptors in rat pituitary and brain', Eur. J. Pharmacol, 46, 377-381, 1997
Gozlan H, El Mestikawy S., Pichat L., Glowinsky J. and Hamon, M., "Identification of presynaptic serotonin autoreceptors using a new ligand 3H-PAT", Nature, 305, 140-142, 1983.
Solomon, Y., Landos, C., Rodbell, M., "A highly selective adenylyl cyclase assay", Anal Biochem, 58, 541-548, 1974
Weiss, S., Sebben, M. and Bockaert, J.J., 'Corticotropin-peptide regulation of intracellular cyclic AMP production in cortical neurons in primary culture', J Neurochem, 45, 869-874, 1985.
Widmark, E.P.M., 'Studies in the concentration of indifferent narcotics in blood and tissues", Acta Med. Scand., 52, 87-164, 1919.

### CITED PATENTS AND PATENT APPLICATIONS:

EP 0 189 612.
EP 0 908 458.

## Claims

**1.** N-oxides of pyridylmethylpiperazine derivatives selected from 1-(2,3-dihydro-1,4-benzodioxin-5-yl)-4-[[5-(4-fluorophenyl)-1-oxido-3-pyridinyl]methyl]-piperazine and 4-(2,3-dihydro-benzo[1,4]dioxin-5-yl-1-[5-4-fluoro-phenyl]-1-oxy-pyridin-3-ylmethyl)-piperazine-1-oxide and tautomers, stereoisomers, pharmacologically acceptable salts, hydrates and solvates thereof.

**3.** The compound according to claims 1 or 2 that is 1-(2,3-dihydro-1,4-benzodioxin-5-yl)-4-[[5-(4-fluorophenyl)-1-oxido-3-pyridinyl]methyl]-piperazine, represented by the formula:

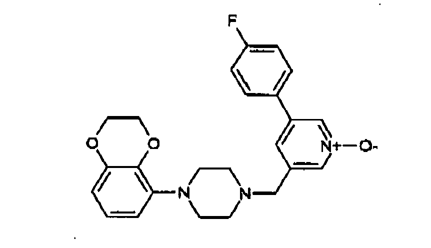

**4.** The compound according to claim 1 that is 4-(2,3-dihydro-benzo[1,4]dioxin-5-yl-1-[5-4-fluorophenyl]-1-oxy-pyridin-3-ylmethyl)piperazine-1-oxide, represented by the formula:

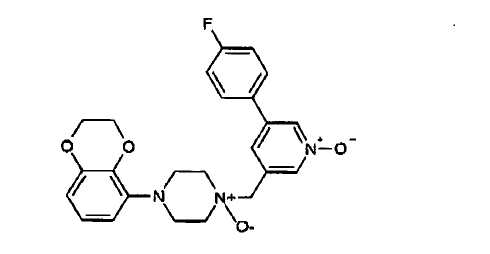

**5.** A medicament, comprising a compound according to any one of the claims 1-4, or a pharmacologically acceptable salt, hydrate or solvate thereof.

**6.** A pharmaceutical composition comprising, apart from a pharmaceutically acceptable carrier and/or at least one pharmaceutically acceptable auxiliary substance, a pharmacologically active amount of at least one compound of any one of the claims 1-4, or a pharmacologically acceptable salt, hydrate or solvate thereof, as an active ingredient.

**7.** Combination pharmaceutical preparation comprising (i) an N-oxide according to any one of claims 1-4; or pharmacologically acceptable salts, hydrates or solvates thereof, and (ii) another therapeutic agent, for simultaneous, separate or sequential use in therapy of Parkinson's disease, aggression, anxiety disorders, autism, vertigo, depression, disturbances of cognition or memory, and in particular schizophrenia, and other psychotic disorders.

**8.** Combination pharmaceutical preparation as claimed in claim 7, wherein said other therapeutic agent is SLV313.

**9.** A compound as claimed in any one of claims 1-4, for treating Parkinson's disease, aggression, anxiety disorders, autism, vertigo, depression, disturbances of cognition or memory, and in particular schizophrenia, and other psychotic disorders.

**10.** Use of a compound as claimed in any one of claims 1-4 for the preparation of a pharmaceutical composition for the treatment of Parkinson's disease, aggression, anxiety disorders, autism, vertigo, depression, disturbances of cognition or memory, and in particular schizophrenia, and other psychotic disorders.

**11.** Use of a combination preparation as claimed in claim 7 to prepare a pharmaceutical composition for treating Parkinson's disease, aggression, anxiety disorders, autism, vertigo, depression, disturbances of cognition or memory, and in particular schizophrenia, and other psychotic disorders.

**12.** Process for the preparation of the compound as claimed in claim 3, wherein pyrifenchloride is oxidized to yield pyrifenchloride-N-oxide, 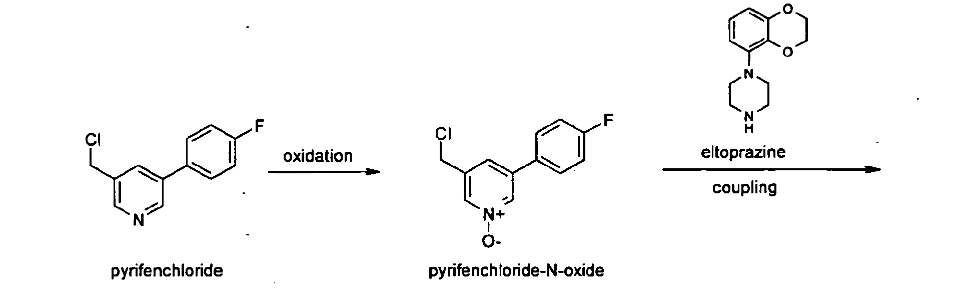 which is coupled with 1-(2,3-dihydro-1,4-benzodioxin-5-yl)-piperazine, also known as eltoprazine, to yield 1-(2,3-dihydro-1,4-benzodioxin-5-yl)-4-[[5-(4-fluorophenyl)-1-oxido-3-pyridinyl]methyl]-piperazine, represented by the formula:

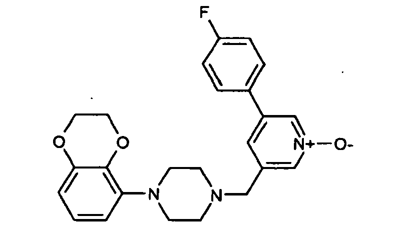

**13.** Process for the preparation of the compound as claimed in claim 4, wherein 1-(2,3-dihydro-1,4-benzodioxin-5-yl)-4-[[5-(4-fluorophenyl)-1-oxido-3-pyridinyl]methyl]-piperazine is oxidized with meta-chloroperbenzoic acid to yield 4-(2,3-dihydro-benzo[1,4]dioxin-5-yl-1-[5-4-fluoro-phenyl]-1-oxy-pyridin-3-ylmethyl)piperazine-1-oxide, represented by the formula:

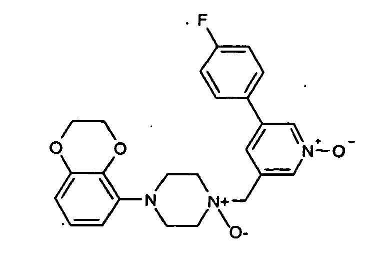

## Patentansprüche

**1.** N-Oxide von Pyridylmethylpiperazin-Derivaten, die unter 1-(2,3-Dihydro-1,4-benzodioxin-5-yl)-4-[[5-(4-fluorophenyl)-1-oxido-3-pyridinyl]methyl]-piperazin und 4-(2,3-Dihydro-benzo[1,4]dioxin-5-yl-1-[5-4-fluoro-phenyl]-1-oxy-pyridin-3-ylmethyl)-piperazin-1-oxid und Tautomeren, Stereoisomeren, pharmakologisch akzeptablen Salzen, Hydraten und Solvaten derselben ausgewählt sind.

**3.** Verbindung nach den Ansprüchen 1 oder 2, die 1-(2,3-Dihydro-1,4-benzodioxin-5-yl)-4-[[5-(4-fluorophenyl)-1-oxido-3-pyridinyl]methyl]-piperazin ist, welche durch die folgende Formel dargestellt ist:

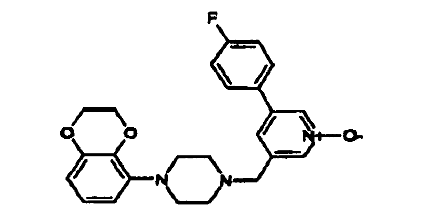

**4.** Verbindung nach Anspruch 1, die 4-(2,3-Dihydro-benzo[1,4]dioxin-5-yl-1-[5-4-fluorophenyl]-1-oxy-pyridin-3-ylmethyl)piperazin-1-oxid ist, welche durch die folgende Formel dargestellt ist:

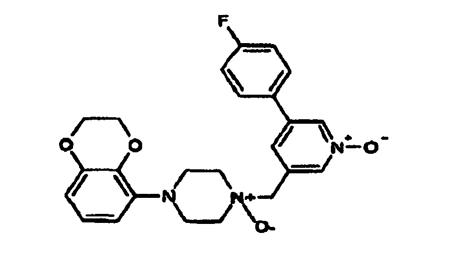

**5.** Medikament, das eine Verbindung nach einem der Ansprüche 1-4 umfasst, oder ein pharmakologisch akzeptables Salz, Hydrat oder Solvat derselben umfasst.

**6.** Pharmazeutische Zusammensetzung, die abgesehen von einem pharmazeutisch akzeptablen Träger und/oder mindestens einer pharmazeutisch akzeptablen Hilfssubstanz eine pharmakologisch aktive Menge von mindestens einer Verbindung nach einem der Ansprüche 1-4 oder ein pharmakologisch akzeptables Salz, Hydrat oder Solvat derselben als Wirkstoff umfasst.

**7.** Kombinierte pharmazeutische Zubereitung, die (I) ein N-Oxid nach einem der Ansprüche 1-4 umfasst, oder pharmakologisch akzeptable Salze, Hydrate oder Solvate derselben, und (II) ein weiteres therapeutisches Mittel zur gleichzeitigen, getrennten oder sequenziellen Verwendung bei der Therapie des Parkinson-Syndroms, Aggression, Angststörungen, Autismus, Schwindel, Depression, Störungen von Kognition oder Gedächtnis und insbesondere Schizophrenie und anderen psychotischen Störungen umfasst.

**8.** Kombinierte pharmazeutische Zubereitung nach Anspruch 7, wobei das andere therapeutische Mittel SLV313 ist.

**9.** Verbindung nach einem der Ansprüche 1-4, zum Behandeln des Parkinson-Syndroms, von Aggression, Angststörungen, Autismus, Schwindel, Depression, Störungen von Kognition oder Gedächtnis und insbesondere Schizophrenie und anderen psychotischen Störungen.

**10.** Verwenden einer Verbindung nach einem der Ansprüche 1-4 zur Herstellung einer pharmazeutischen Zusammensetzung zur Behandlung des Parkinson-Syndroms, von Aggression, Angststörungen, Autismus, Schwindel, Depression, Störungen von Kognition oder Gedächtnis und insbesondere Schizophrenie und anderen psychotischen Störungen.

**11.** Verwendung einer Kombinationszubereitung nach Anspruch 7 zum Herstellen einer pharmazeutischen Zusammensetzung zum Behandeln des Parkinson-Syndroms, von Aggression, Angststörungen, Autismus, Schwindel, Depression, Störungen von Kognition oder Gedächtnis und insbesondere Schizophrenie und anderen psychotischen Störungen.

**12.** Verfahren zur Herstellung der Verbindung nach Anspruch 3, wobei Pyrifenchlorid oxidiert wird, um Pyrifenchlorid-N-Oxid zu erhalten, 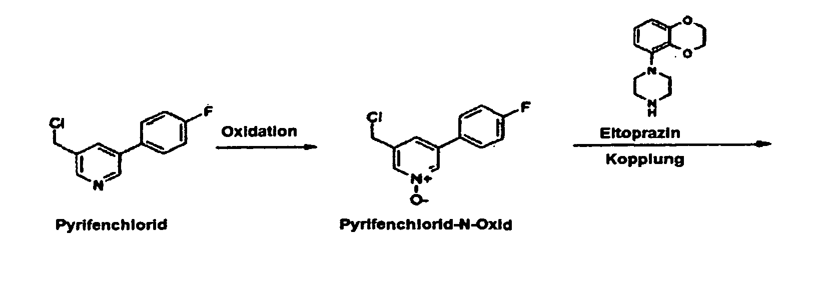 welches mit 1-(2,3-Dihydro-1,4-benzodioxin-5-yl)-piperazin gekoppelt wird, das auch als Eltoprazin bekannt ist, um 1-(2,3-Dihydro-1,4-benzodioxin-5-yl)-4-[[5-(4-fluorophenyl)-1-oxido-3-pyridinyl]methyl]-piperazin zu ergeben, das durch die folgende Formel dargestellt ist:

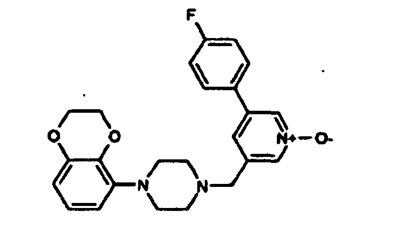

**13.** Verfahren zur Herstellung der Verbindung nach Anspruch 4, wobei 1-(2,3-Dihydro-1,4-benzodioxin-5-yl)-4-[[5-(4-fluorophenyl)-1-oxido-3-pyridinyl]methyl]piperazin oxidiert wird, um 4-(2,3-Dihydrobenzo[1,4]dioxin-5-yl-1-[5-4-fluoro-phenyl]-1-oxy-pyridin-3-ylmethyl)piperazin-1-oxid zu ergeben, welches durch die folgende Formel dargestellt ist:

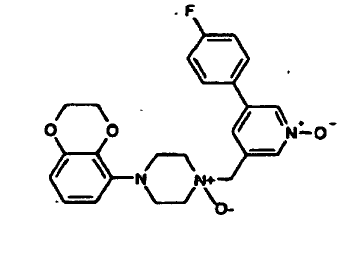

## Revendications

**1.** N-oxydes de dérivés de pyridylméthylpipérazine choisis parmi la 1-(2,3-dihydro-1,4-benzodioxin-5-yl)-4-[[5-(4-fluorophényl)-1-oxydo-3-pyridinyl]méthyl]pipérazine et le 4-(2,3-dihydro-benzo[1,4]dioxin-5-yl-1-[5-4-fluoro-phényl]-1-oxy-pyridin-3-ylméthyl)pipérazine-1-oxyde et leurs tautomères, stéréoisomères, sels pharmacologiquement acceptables, hydrates et solvates.

**3.** Composé selon la revendication 1 ou 2 qui est la 1-(2,3-dihydro-1,4-benzodioxin-5-yl)-4-[[5-(4-fluorophényl)-1-oxydo-3-pyridinyl]-méthyl]-pipérazine représentée par la formule :

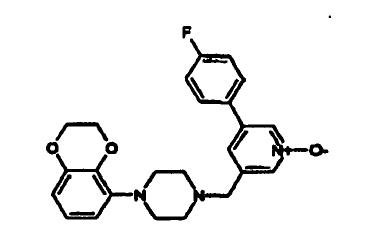

**4.** Composé selon la revendication 1 qui est le 4-(2,3-dihydro-benzo[1,4]dioxin-5-yl-1-[5-4-fluorophényl]-1-oxy-pyridin-3-ylméthyl)-pipérazine-1-oxyde représenté par la formule :

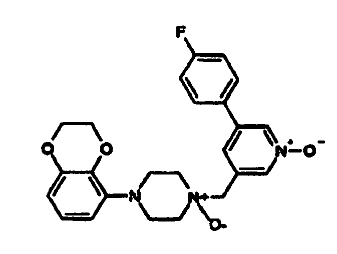

**5.** Médicament comprenant un composé selon l'une quelconque des revendications 1-4, ou un sel, hydrate ou solvate pharmacologiquement acceptable de celui-ci.

**6.** Composition pharmaceutique comprenant, mis à part un vecteur pharmaceutiquement acceptable et/ou au moins une substance auxiliaire pharmaceutiquement acceptable, une quantité pharmacologiquement active d'au moins un composé selon l'une quelconque des revendications 1-4, ou d'un sel, hydrate ou solvate pharmacologiquement acceptable de celui-ci, comme ingrédient actif.

**7.** Préparation pharmaceutique de type association comprenant (i) un N-oxyde selon l'une quelconque des revendications 1-4 ; ou des sels, hydrates ou solvates pharmacologiquement acceptables de celui-ci, et (ii) un autre agent thérapeutique, pour l'utilisation simultanée, séparée ou successive dans la thérapie de la maladie de Parkinson, de l'agression, des troubles d'anxiété, de l'autisme, du vertige, de la dépression, des perturbations de la cognition ou de la mémoire, et en particulier de la schizophrénie, et d'autres troubles psychotiques.

**8.** Préparation pharmaceutique de type association selon la revendication 7, où ledit autre agent thérapeutique est SLV313.

**9.** Composé selon l'une quelconque des revendications 1-4, pour traiter la maladie de Parkinson, l'agression, les troubles d'anxiété, l'autisme, le vertige, la dépression, les perturbations de la cognition ou de la mémoire, et en particulier la schizophrénie, et d'autres troubles psychotiques.

**10.** Utilisation d'un composé selon l'une quelconque des revendications 1-4 pour la préparation d'une composition pharmaceutique pour le traitement de la maladie de Parkinson, de l'agression, des troubles d'anxiété, de l'autisme, du vertige, de la dépression, des perturbations de la cognition ou de la mémoire, et en particulier de la schizophrénie, et d'autres troubles psychotiques.

**11.** Utilisation d'une préparation de type association selon la revendication 7 pour préparer une composition pharmaceutique pour traiter la maladie de Parkinson, l'agression, les troubles d'anxiété, l'autisme, le vertige, la dépression, les perturbations de la cognition ou de la mémoire, et en particulier la schizophrénie, et d'autres troubles psychotiques.

**12.** Procédé pour la préparation du composé selon la revendication 3, où le chlorure de pyrifène est oxydé pour donner le N-oxyde de chlorure de pyrifène 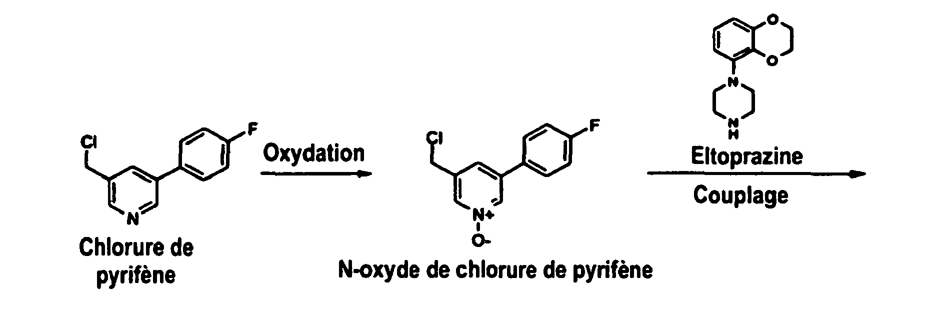 qui est couplé avec la 1-(2,3-dihydro-1,4-benzodioxin-5-yl)-pipérazine, connue également comme étant l'eltoprazine, pour donner la 1-(2,3-dihydro-1,4-benzodioxin-5-yl)-4-[[5-(4-fluorophényl)-l-oxydo-3-pyridinyl]-méthyl]-pipérazine, représentée par la formule :

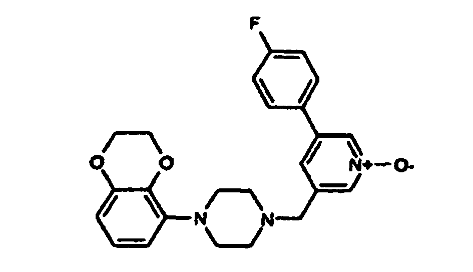

**13.** Procédé pour la préparation du composé selon la revendication 4, où la 1-(2,3-dihydro-1,4-benzodloxin-5-yl)-4-[[5-(4-fluorophényl)-1-oxydo-3-pyridinyl]méthyl]pipérazine est oxydée avec l'acide méta-chloroperbenzoïque pour donner le 4-(2,3-dihydrobenzo[1,4]dioxin-5-yl-1-[5-4-fluoro-phényl]-1-oxy-pyridin-3-ylméthyl)pipérazine-1-oxyde, représenté par la formule :
